**Europäisches Patentamt**

**European Patent Office**

**Office européen des brevets**

(19)

(11) **EP 1 375 507 A1**

(12) **EUROPÄISCHE PATENTANMELDUNG**

| | |
|---|---|
| (43) Veröffentlichungstag:<br>**02.01.2004 Patentblatt 2004/01** | (51) Int Cl.⁷: **C07H 1/06**, C07H 7/00 |

(21) Anmeldenummer: **03011164.5**

(22) Anmeldetag: **26.05.2003**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **20.06.2002 DE 10227432**

(71) Anmelder: **SÜDZUCKER AKTIENGESELLSCHAFT MANNHEIM/OCHSENFURT 68165 Mannheim (DE)**

(72) Erfinder:
• **Begli, Alireza Haji Dr.**
 **67305 Ramsen (DE)**

• **Hirth, Joachim**
 **64823 Gross-Umstadt (DE)**
• **Kowalczyk, Jörg Dr.**
 **67304 Eisenberg/Steinborn (DE)**
• **Kunz, Markwart Dr.Prof.**
 **67550 Worms (DE)**
• **Vogel, Herbert Dr.Prof.**
 **67069 Ludwigshafen (DE)**

(74) Vertreter: **Schrell, Andreas, Dr. et al Gleiss & Grosse Leitzstrasse 45 70469 Stuttgart (DE)**

(54) **Verfahren zur Trennung von Kohlenhydratcarbonsäuren**

(57) Die vorliegende Erfindung betrifft ein Verfahren zur Trennung von Kohlenhydratcarbonsäuren aus ihrem Gemisch durch eine mindestens einstufige Reaktivextraktion mit einem Extraktionsmittelsystem aus einem $C_{>3}$-Alkohol, insbesondere einem $C_4$-$C_8$-Alkohol, und mindestens einem Alkylamin.

Figur

EP 1 375 507 A1

Printed by Jouve, 75001 PARIS (FR)

**Beschreibung**

Die vorliegende Erfindung betrifft ein Verfahren zur Trennung einer Kohlenhydratcarbonsäure aus einem Gemisch dieser Kohlenhydratcarbonsäure und mindestens einer anderen Kohlenhydratcarbonsäure.

Kohlenhydratcarbonsäuren, darunter die Oxidationsprodukte der Monosaccharide wie Aldonsäuren, Aldarsäuren oder Uronsäuren, finden beispielsweise Verwendung als milde Reduktionsmittel (reduzierende Zucker) in chemischen Verfahren, als milde Säuren, beispielsweise in Metallbeizmitteln, Textilhilfsmitteln, zur Herstellung sauer schmeckender Lebens-, Genussmittel oder Getränke, als Anion von Wirkstoff-Salzen in Arzneimittelzubereitungen oder als Komplexbildner für Metallionen.

Ihre Herstellung erfolgt hauptsächlich durch mikrobielle Oxidation von Monosacchariden in einem Fermentationsmedium, worin sie nach Abschluß der enzymatischen Fermentation in Form einer verdünnten wässrigen Lösung vorliegen.

Aufgrund der guten Wasserlöslichkeit der Kohlenhydratcarbonsäuren können zur Extraktion einer Kohlenhydratcarbonsäure aus ihren verdünnten wässrigen Lösungen im Stand der Technik meist Reaktivextraktionen eingesetzt werden, bei denen einem organischem Extraktionsmittel wasserunlösliche, höhere 2-oder 3-wertige Amine zugesetzt werden, die mit der Kohlenhydratcarbonsäure das entsprechende Ammoniumsalz bilden, welches deutlich besser aus der wässrigen Phase extrahiert werden kann.

In diesem Zusammenhang beschreibt die DE 195 22 377 A1 ein Verfahren zur Gewinnung von unter anderem einer Kohlenhydratcarbonsäure aus ihrer wässrigen Lösung mittels Reaktivextraktion und anschließender Destillation des erhaltenen Extrakts, indem als Extraktionsmittel ein sekundäres Amid eingesetzt wird. Darüber hinaus beschreibt die DE 197 47 790 C1 ein kontinuierliches Verfahren zur Aufkonzentrierung von verdünnten wässrigen Lösungen von unter anderem einer Kohlenhydratcarbonsäure, wobei die Kohlenhydratcarbonsäure mit einem Extraktionsmittelsystem bestehend aus einem organischen Lösungsmittel und einem Alkylamin aus der verdünnten wässrigen Lösung extrahiert, aus dem organischen Extrakt das Lösungsmittel abdestilliert und anschließend die Kohlenhydratcarbonsäure als wasserlösliches Salz einer wässrigen Alkylamin-Lösung aus dem erhaltenen Rückstand gewonnen wird.

Mit den vorgenannten Verfahren lassen sich mittels optimierter organischer Extraktionsmittelsysteme Kohlenhydratcarbonsäuren aus wässrigen Lösungen isolieren. Nachteilig dabei ist, dass die in wässrigen Lösungen, insbesondere in Fermentationsmedien, gleichzeitig als Gemisch enthaltenen verschiedenen Kohlenhydratcarbonsäuren nicht getrennt extrahiert werden können. Die Fraktionierung dieser gemeinsam vorkommenden Kohlenhydratcarbonsäuren ist bislang nur durch den Einsatz von chromatographischen Trennmethoden möglich. Hierbei werden insbesondere Systeme mit sauren und/oder basischen Ionenaustauschersäulen eingesetzt.

Nachteilig an chromatographischen Trennsystemen ist jedoch, dass die Ionenaustauscher in diesen Systemen wiederkehrende Regenerationsphasen benötigen, so dass parallele Systeme oder semikontinuierliche Arbeitsweisen erforderlich sind und eine kontinuierliche Trennung -wie im großtechnischen Maßstab erwünscht- mit diesen herkömmlichen Trennsystemen nicht möglich ist. Kommen darüber hinaus als wässrige Lösungen der Kohlenhydratcarbonsäuren Fermentationsmedien oder andere mit Edukten oder weiteren Nebenprodukten kontaminierte Mutterlaugen zum Einsatz, so wird die chromatographische Trennoperation erschwert oder gar unmöglich gemacht. In diesem Fall sind zusätzliche vorgeschaltete Aufreinigungsschritte notwendig, was die verfahrenstechnische Darstellung der Kohlenhydratcarbonsäuren weiter kompliziert und verteuert.

Demgemäß ist es Aufgabe der vorliegenden Erfindung, ein verbessertes Verfahren zur Extraktion von Kohlenhydratcarbonsäuren aus wässriger Lösung bereitzustellen, das gleichzeitig eine einfache und kostengünstige Trennung verschiedener Kohlenhydratcarbonsäuren, die als Gemisch in der wässrigen Lösung vorliegen, ermöglicht.

Diese Aufgabe wird dadurch gelöst, dass ein Verfahren zur Trennung einer ersten Kohlenhydratcarbonsäure aus einem Gemisch dieser Kohlenhydratcarbonsäure und mindestens einer weiteren zweiten Kohlenhydratcarbonsäure bereitgestellt wird, wobei diese erste Kohlenhydratcarbonsäure in einem Reaktivextraktionsverfahren mit mindestens einer Stufe unter Verwendung eines organischen Extraktionsmittelsystems, das mindestens einen Alkohol mit einem mehr als drei C-Atome aufweisenden Kohlenstoffskelett, das heißt $C_{>3}$-Alkohol, insbesondere einen $C_4$-$C_8$-Alkohol, und mindestens ein Alkylamin enthält, von der mindestens einen weiteren Kohlenhydratcarbonsäure aus dem Gemisch abgetrennt wird. Erfindungsgemäß bevorzugt sind die Kohlenhydratcarbonsäuren oxidierter Monosaccharide, nämlich Monosaccharidcarbonsäuren, insbesondere die Glucoseoxidationsprodukte D-Glucuronsäure, D-Gluconsäure oder D-Glucarsäure. Erfindungsgemäß bevorzugt wird als organisches Extraktionsmittelsystem ein $C_4$-Alkohol, besonders bevorzugt 1-Butanol, zusammen mit einem tertiären Trialkylamin, besonders bevorzugt Tri-n-Octylamin, eingesetzt.

Es wurde überraschenderweise gefunden, dass $C_{>3}$-Alkohole, insbesondere $C_4$-$C_8$-Alkohole, insbesondere 1-Butanol, die Extraktionseigenschaften von Trialkylaminen, insbesondere Tri-n-Octylamin, in Form der Parameter Verteilungskoeffizient und Selektivität erheblich verbessern. Besonders überraschend war dabei, dass eine große Abstufung in den Verteilungskoeffizienten der Kohlenhydratcarbonsäuren, insbesondere von Glucuron-, Glucon- und Glucarsäure, auftrat. Die dabei erhaltenen Trennfaktoren lassen vorteilhafterweise die erfindungsgemäße Fraktionierung der vorgenannten Kohlenhydratcarbonsäuren in der Extraktion zu. Erfindungsgemäß bevorzugt werden so nach dem Prinzip der multiplikativen Verteilung über eine Vielstufenextraktion, in Abhängigkeit von der Zahl der Verteilungselemente, direkt aus der Reaktivreaktion die mindestens zwei im Gemisch vorhandenen Kohlenhydratcarbonsäuren in den organischen Extrakten der einzelnen Verteilungselemente fraktioniert erhalten.

Somit erlaubt das erfindungsgemäße Verfahren vorteilhaft die Trennung und Isolierung mindestens einer Kohlenhydratcarbonsäure aus einem Gemisch dieser Kohlenhydratcarbonsäure und mindestens einer weiteren Kohlenhydratcarbonsäure, insbesondere aus einer wässrigen Lösung, einer Mutterlauge oder einem Fermentationsmedium, in einem einheitlichen Reaktivextraktionsverfahren. Das erfindungsgemäße Verfahren vermeidet damit, dass im Anschluss an die Extraktion noch ein zusätzliches und aufwendiges Trennverfahren zur Trennung der im Gemisch vorhandenen Kohlenhydratcarbonsäuren durchgeführt werden muss. Das erfindungsgemäße Verfahren stellt somit eine besonders einfache und kostengünstige Lösung zur Darstellung von Kohlenhydratcarbonsäuren aus wässrigen Lösungen, insbesondere Mutterlaugen oder Fermentationsmedien, dar.

Durch den Einsatz der erfindungsgemäßen Reaktivextraktion können außerdem Kohlenhydratcarbonsäuren aus deren Gemischen auch in stark verdünnten wässrigen Lösungen isoliert und separiert werden.

Im Zusammenhang mit der vorliegenden Erfindung wird unter dem Begriff "Reaktivextraktion" ein Verfahren der Flüssig-Flüssig-Extraktion bezeichnet, bei dem die zu extrahierende Verbindung durch Reaktion mit einem im Extraktionsmittel enthaltenen Stoff aus der wässrigen Phase in die organische Extrakt-Phase überführt wird. Diese Extraktion lässt sich kontinuierlich oder diskontinuierlich durchführen. In der technischen Anwendung wird die kontinuierliche Extraktion im Gegenstromverfahren bevorzugt.

Im Zusammenhang mit der vorliegenden Erfindung wird unter dem Begriff "Verteilungskoeffizient" das Verhältnis des Anteils der Kohlenhydratcarbonsäure in Gew.-% im Extrakt zum Anteil der Kohlenhydratcarbonsäure in Gew.-% in der wässrigen Phase verstanden.

Im Zusammenhang mit der vorliegenden Erfindung wird unter dem Begriff "Trennfaktor"(T) der Quotient der Verteilungskoeffizienten zweier Kohlenhydratcarbonsäuren im Gemisch in der wässrigen Phase verstanden, wobei der Verteilungskoeffizient mit dem zahlenmäßig größeren Wert durch den Verteilungskoeffizient mit dem zahlenmäßig kleineren Wert dividiert wird.

$$K_{Säure_i} = \frac{[Säure_i]_{org.}}{[Säure_i]_{Wasser}}$$

$$T = \frac{K_{Säure\ 1}}{K_{Säure\ 2}};\ K_{Säure\ 1} > K_{Säure\ 2}$$

Im Zusammenhang mit der vorliegenden Erfindung wird unter dem Begriff "Selektivität" ($S_{Säure\ /H_2O}$) das Verhältnis des Quotienten Kohlenhydratcarbonsäure zu Wasser ($H_2O$) im organischen Extrakt *(org.)* zum Quotienten Kohlenhydratcarbonsäure zu Wasser in der wässrigen Phase verstanden. Dabei gehen in die Rechnung die Anteile der Substanzen in Gew.-% ein.

$$K_{H_2O} = \frac{[H_2O]_{org.}}{[H_2O]_{H_2O}}$$

$$S_{Säure\ /\ H_2O} = \frac{K_{säure}}{K_{H_2O}}$$

In einer besonderen Ausführungsform des erfindungsgemäßen Verfahrens sind die zu trennenden Kohlenhydratcarbonsäuren Carbonsäuren von Disacchariden, die eine säureempfindliche glycosidische Bindung aufweisen. Solche Disaccharidcarbonsäuren sind insbesondere Saccharosemonocarbonsäuren. Mit den erfindungsgemäßen Verfahren ist insbesondere die schonende Trennung säureempfindlicher Kohlenhydratcarbonsäuren, beispielsweise der Saccharosemonocarbonsäure, die eine säurempfindliche glycosidische Bindung beinhaltet, möglich.

In einer weiteren bevorzugten Ausführungsform des erfindungsgemäßen Verfahrens sind die zu trennenden Kohlenhydratcarbonsäuren Monosaccharidcarbonsäuren, die durch selektive Oxidation von Monosacchariden als Mono- oder Dicarbonsäuren entstehen. Insbesondere sind dies Aldonsäuren, wie die Alcurinsäure, Aldarsäuren, wie die Galactarsäure, die (+)-Weinsäure oder die *meso*-Weinsäure, sowie Uronsäuren wie die Galacturonsäure, die Guluronsäure, die Iduronsäure oder die Manuronsäure.

In einer bevorzugten Ausführungsform des erfindungsgemäßen Verfahrens wird die Reaktivextraktion als eine kontinuierliche Vielstufenextraktion durchgeführt, die nach dem Prinzip der multiplikativen Verteilung insbesondere verfahrenstechnischer Art und im großtechnischen Maßstab durchgeführt wird. Erfindungsgemäß bevorzugt ist dabei die Verwendung mehrstufiger Extraktionsapparate, beispielsweise Batterien von Mischern oder Scheidern oder Füllkörperkolonnen, die in an sich bekannter Weise eingesetzt werden. Durch die überraschenderweise gefundenen deutlichen Abstufungen der Verteilungskoeffizienten der einzelnen Kohlenhydratcarbonsäuren im Gemisch mit dem erfindungsgemäßen Extraktionsmittelsystem bleibt -besonders vorteilhaft- die Zahl der notwendigen Trennstufen zur Trennung und Isolation der im Gemisch vorhandenen Kohlenhydratcarbonsäuren gering.

In einer Variante ist bei der kontinuierlichen Vielstufenextraktion erfindungsgemäß eine integrierte Lösungsmittelrückgewinnung aus der organischen Extrakt-Phase vorgesehen. Insbesondere wird dabei die integrierte Lösungsmittelrückgewinnung nach dem Verfahren der Destillation, insbesondere der Rektifikation bevorzugt im großtechnischen Maßstab durchgeführt. Bevorzugt wird ein Rektifikationsschritt, besonders bevorzugt werden mehrere Rektifikationsschritte durchgeführt, um das organische Lösungsmittel aus der Extrakt-Phase zurückzugewinnen. Durch den Einsatz des erfindungsgemäß bevorzugten mehrstufigen Extraktionsprozesses mit einer integrierten Lösungsmittelrückgewinnung ist ein ökonomischer und kontinuierlicher Trennprozess realisierbar.

In einer bevorzugten Ausführungsform des erfindungsgemäßen Verfahrens liegt die mindestens eine Kohlenhydratcarbonsäure in einer verdünnten wässrigen Lösung, insbesondere in einer Mutterlauge oder einem Fermentationsmedium vor. Dabei beträgt der Anteil der mindestens einen Kohlenhydratcarbonsäure in der verdünnten wässrigen Lösung bezogen auf Wasser 0,5 bis 25 Gew.-%, bevorzugt 1 bis 8 Gew.-%, besonders bevorzugt 5 Gew.-%.

Erfindungsgemäß bevorzugt beträgt die Temperatur während der Reaktivextraktion 0 bis 100°C, bevorzugt 20 bis 60°C, besonders bevorzugt 40°C.

Erfindungsgemäß bevorzugt wird das Molverhältnis (Molenbruch) der im Gemisch vorhandenen Kohlenhydratcarbonsäuren und Alkylamin durch Variation des Anteils des Alkylamins im organischen Extraktionsmittelsystem von 0,3 bis 5,0, bevorzugt von 1,5 bis 2,5, besonders bevorzugt auf 2 eingestellt.

Erfindungsgemäß bevorzugt beträgt das Masse-Verhältnis der wässrigen Lösung der mindestens einen Kohlenhydratcarbonsäure und dem organischen Extraktionsmittelsystem zu Beginn der Reaktivreaktion 0,5 bis 2, bevorzugt 0,7 bis 1,4, besonders bevorzugt 1.

Weitere vorteilhafte Ausführungen der Erfindung ergeben sich aus den Unteransprüchen.

Weitere vorteilhafte Ausführungsformen des erfindungsgemäßen Verfahrens sind auch den folgenden Ausführungsbeispielen und der dazugehörigen Figur zu entnehmen:

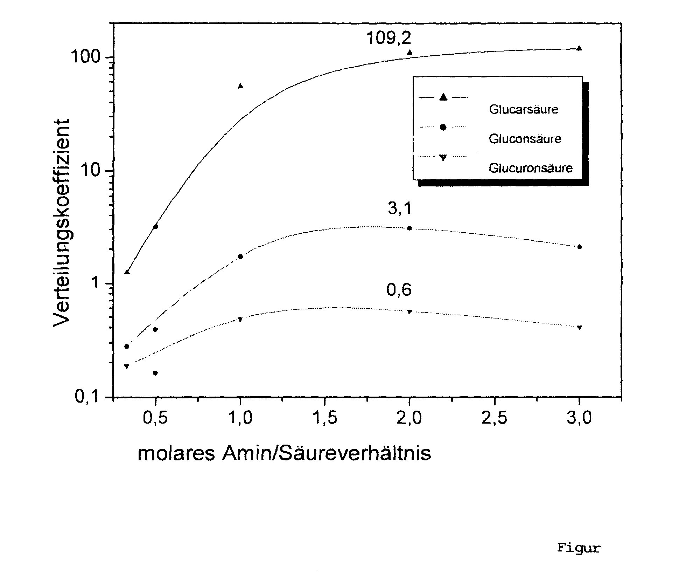

Die Figur stellt dar: Die individuellen Verteilungskoeffizienten der Kohlenhydratcarbonsäuren D-Glucuron-, D-Glucon- und D-Glucarsäure als Gemisch in wässriger Lösung bei Verwendung eines Extraktionsmittelsystems bestehend aus 1-Butanol und Tri-n-Octylamin als Funktion des molaren Verhältnisses von Tri-n-Octylamin zu allen im Gemisch vorhandenen Kohlenhydratcarbonsäuren. Die weiteren Parameter ergeben sich aus Beispiel 2.

Beispiel 1:

Extraktion von Glucuron-, Glucon- oder Glucarsäure aus einer wässrigen Lösung

Zur Bestimmung des Verteilungskoeffizienten der Kohlenhydratcarbonsäuren D-Glucuronsäure, D-Gluconsäure und D-Glucarsäure in Abhängigkeit des Anteils an Tri-n-Octylamin im organischen Extraktionsmittelsystem werden jeweils wässrige Lösungen der Kohlenhydratcarbonsäuren mit einer Anfangskonzentration von 5 Gew.-% eingesetzt.

Zur Extraktion werden gleiche Masseanteile organische und wässrige Phase eingesetzt. Die Extraktionstemperatur beträgt 40 °C. Durch Variation des Anteils an Tri-n-Octylamin in der organischen Phase wird das molare Amin/ Säure-Verhältnis eingestellt.

Protokoll: 20 g einer wässrigen Lösung der entsprechenden Kohlenhydratcarbonsäure werden mit 20 g Extraktionsmittel bei 40 °C bis zur Einstellung des Gleichgewichts gerührt und das Gemisch anschließend stehen gelassen, bis sich die organische von der wässrigen Phase klar getrennt hat.

Durch HPLC-Analyse wird die Konzentration der in der wässrigen Phase verbleibenden Kohlenhydratcarbonsäure ermittelt und der Verteilungskoeffizient berechnet.

Ergebnis:

| Kohlenhydratsäure | v | $K_{Säure}$ | $S_{Säure/Wasser}$ |
|---|---|---|---|
| D-Glucuronsäure | 0,33 | 0,38 | 1,79 |
| | 0,51 | 0,47 | 1,95 |
| | 1,00 | 0,45 | 2,55 |
| | **2,00** | **0,35** | **2,06** |
| | 3,01 | 0,26 | 2,41 |
| D-Gluconsäure | 0,34 | 0,49 | 1,78 |
| | 0,57 | 0,91 | 3,28 |
| | 1,10 | 2,29 | 9,13 |
| | **2,19** | **3,44** | **17,49** |
| | 3,27 | 2,69 | 14,68 |
| | 5,42 | 1,63 | 10,97 |
| D-Glucarsäure | 0,30 | 0,50 | 2,12 |
| | 0,51 | 0,89 | 3,82 |
| | 1,00 | 3,19 | 10,12 |
| | **1,99** | **54,32** | **147,19** |
| | 2,99 | 68,51 | 202,62 |
| v = molares Amin/Säure-Verhältnis, $K_{Säure}$ = Verteilungskoeffizient, $S_{Säure/Wasser}$ = Selektivität | | | |

Bei einem molaren Amin/Säure-Verhältnis von cirka 2 unterscheidet sich der Verteilungskoeffizient der Gluconsäure-Extraktion von dem der Glucuronsäure-Extraktion etwa um den Trennfaktor 10. Der Trennfaktor der Glucarsäure-Extraktion bezogen auf die Gluconsäure-Extraktion beträgt dabei cirka 16. Der Trennfaktor der Glucarsäure-Extraktion und der Glucuronsäure-Extraktion beträgt cirka 155.

Die einzelnen Trennfaktoren der untersuchten Kohlenhydratcarbonsäuren unterscheiden sich somit mindestens um ungefähr eine Größenordnung.

Beispiel 2:

Trennung eines wässrigen Gemisches aus D-Glucuron-, D-Glucon- und D-Glucarsäure

Abweichend von Beispiel 1, wo die Verteilungskoeffizienten dieser Kohlenhydratcarbonsäuren jeweils in ihren Reinlösungen untersucht werden, werden hier die individuellen Verteilungskoeffizienten der Kohlenhydratcarbonsäuren bestimmt, wenn diese gemeinsam in einem Gemisch in wässriger Lösung vorliegen.

Im Einzelnen betragen dabei die Gewichtsanteile der drei Kohlenhydratcarbonsäuren in der wässrigen Lösung jeweils 1,6 Gew.-%, so dass sich eine Gesamtsäurekonzentration von cirka 5 Gew.-% ergibt. Als Extraktionsmittelsystem dient das erfindungsgemäße Gemisch aus 1-Butanol und Tri-n-Octylamin. Die Extraktionstemperatur liegt bei 40°C.

Es werden jeweils gleiche Masseanteile organische und wässrige Phase eingesetzt. Durch Variation des Anteils an Tri-n-Octylamin in der organischen Phase wird das molare Amin/Säure-Verhältnis eingestellt.

Das Protokoll entspricht Beispiel 1.

Ergebnis:

Die individuellen Verteilungskoeffizienten von D-Glucarsäure, D-Gluconsäure und D-Glucuronsäure in Abhängigkeit vom molaren Amin/Säure-Verhältnis ist in der Figur dargestellt. Im Vergleich zu Beispiel 1 treten dabei bei einem molaren Amin/Säure-Verhältnis von cirka 2 folgende Verteilungskoeffizienten auf.

| Kohlenhydratsäure | $K_{Säure}$ |
|---|---|
| D-Glucuronsäure | 0,6 |
| D-Gluconsäure | 3,1 |
| D-Glucarsäure | 109 |

Überraschenderweise ergibt sich, dass mit dem erfindungsgemäß eingesetzten Extrationsmittelsystem individuelle Verteilungskoeffizienten der Kohlenhydratcarbonsäuren im Gemisch erhalten werden, die eine Fraktionierung dieser Kohlenhydratcarbonsäuren mittels einer Reaktivextraktion in einer sehr geringen Anzahl notwendiger Trennstufen ermöglichen. Die Verteilungskoeffizienten liegen dabei überraschenderweise in den gleichen Größenbereichen wie bei den Extraktionsexperimenten der Einzelsubstanzen.

Im Falle der D-Glucarsäure ist darüber hinaus eine weitere Zunahme des Verteilungskoeffizienten zu beobachten.

Die Trennfaktoren einer extraktiven Gleichgewichtsstufe betragen bei einem molaren Amin/Säure-Verhältnis von cirka 2:

| Kohlenhydratsäure | Trennfaktor |
|---|---|
| D-Glucuronsäure zu D-Gluconsäure | 5,2 |
| D-Gluconsäure zu D-Glucarsäure | 35,2 |
| D-Glucuronsäure zu D-Glucarsäure | 182 |

## Patentansprüche

1.  Verfahren zur Trennung einer Kohlenhydratcarbonsäure aus einem Gemisch dieser Kohlenhydratcarbonsäure und mindestens einer anderen Kohlenhydratcarbonsäure, wobei die Kohlenhydratcarbonsäure in einer mindestens einstufigen Reaktivextraktion mit einem Extraktionsmittelsystem enthaltend mindestens einen $C_{>3}$-Alkohol und mindestens ein Alkylamin von der mindestens einen anderen Kohlenhydratcarbonsäure im Gemisch aus diesem abgetrennt wird.

2.  Verfahren nach Anspruch 1, wobei die Kohlenhydratcarbonsäuren Monosaccharidcarbonsäuren sind.

3.  Verfahren nach Anspruch 2, wobei die Kohlenhydratcarbonsäuren Glucoseoxidationsprodukte ausgewählt aus der Gruppe bestehend aus D-Glucuronsäure, D-Gluconsäure und D-Glucarsäure sind.

4.  Verfahren nach einem der vorangehenden Ansprüche, wobei der $C_{>3}$-Alkohol ein $C_4$-$C_8$-Alkohol, insbesondere ein $C_4$-Alkohol, bevorzugt 1-Butanol, ist.

5.  Verfahren nach einem der vorangehenden Ansprüche, wobei das Alkylamin ein tertiäres Trialkylamin, bevorzugt Tri-n-octylamin, ist.

6.  Verfahren nach einem der vorangehenden Ansprüche, wobei die Kohlenhydratcarbonsäuren Disaccharidcarbonsäuren sind, die eine säurempfindliche glycosidische Bindung beinhalten.

7.  Verfahren nach Anspruch 6, wobei die Disaccharidcarbonsäuren Saccharosemonocarbonsäuren sind.

8.  Verfahren nach einem der vorangehenden Ansprüche, wobei die Reaktivextraktion in einer kontinuierlichen Vielstufenextraktion durchgeführt wird.

9.  Verfahren nach Anspruch 8, wobei bei der kontinuierlichen Vielstufenextraktion eine integrierte Lösungsmittelrückgewinnung aus der Extraktphase vorgesehen ist.

10. Verfahren nach Anspruch 9, wobei zur integrierten Lösungsmittelrückgewinnung mindestens ein Rektifikationsschritt durchgeführt wird.

**11.** Verfahren nach einem der vorangehenden Ansprüche, wobei die Kohlenhydratcarbonsäuren im Gemisch in einer verdünnten wässrigen Lösung vorliegen.

**12.** Verfahren nach Anspruch 11, wobei der Anteil der Kohlenhydratcarbonsäuren im Gemisch in der verdünnten wässrigen Lösung von 0,5 bis 25 Gew.-% bezogen auf Wasser, bevorzugt von 1 bis 8 Gew.-% beträgt.

**13.** Verfahren nach einem der vorangehenden Ansprüche, wobei die Temperatur bei der Reaktivextraktion von 0 bis 100°C, bevorzugt von 20 bis 60°C beträgt.

**14.** Verfahren nach einem der vorangehenden Ansprüche, wobei das Mol-Verhältnis von Kohlenhydratcarbonsäure und Alklyamin von 0,3 bis 5,0, bevorzugt von 1,5 bis 2,5 beträgt.

**15.** Verfahren nach einem der vorangehenden Ansprüche, wobei das Masse-Verhältnis der wässrigen Lösung der Kohlenhydratcarbonsäuren und dem Extraktionsmittelsystem zu Beginn der Reaktivextraktion von 0,5 bis 2, bevorzugt von 0,7 bis 1,4 beträgt.

Figur

EP 1 375 507 A1

Nummer der Anmeldung

EP 03 01 1164

**Europäisches Patentamt**

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | EP 0 359 042 A (BASF AG) 21. März 1990 (1990-03-21) Seite 2, Zeile 17 Seite 4, Zeile 8- Zeile 17 Seite 5, Zeile 14- Zeile 21 --- | 1,2,4,5, 8-15 | C07H1/06 C07H7/00 |
| A | J. HIRTH ET AL.: "Isolierung von Kohlenhydratcarbonsäuren aus wässrigen Lösungen durch Reaktivextraktion" CHEM. ING. TECH., Bd. 74, 2002, Seiten 345-350, XP002258408 * das ganze Dokument * --- | 1 | |
| P,X | J. HIRTH ET AL.: "Trennung von Glucoseoxigenaten durch Reaktivextraktion" CHEM. ING. TECH., Bd. 75, 2003, Seiten 294-298, XP002258409 * das ganze Dokument * ----- | 1-15 | |

**RECHERCHIERTE SACHGEBIETE (Int.Cl.7)**

C07H

Der vorliegende Recherchenbericht wurde für alle Patentansprüche erstellt

| Recherchenort | Abschlußdatum der Recherche | Prüfer |
|---|---|---|
| DEN HAAG | 20. Oktober 2003 | de Nooy, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 03 01 1164

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-10-2003

| Im Recherchenbericht angeführtes Patentdokument | Datum der Veröffentlichung | Mitglied(er) der Patentfamilie | Datum der Veröffentlichung |
|---|---|---|---|
| EP 0359042 A | 21-03-1990 | DE 3831070 A1<br>DE 58908961 D1<br>EP 0359042 A2<br>JP 2121947 A | 22-03-1990<br>16-03-1995<br>21-03-1990<br>09-05-1990 |

EPO FORM P0461

Für nähere Einzelheiten zu diesem Anhang : siehe Amtsblatt des Europäischen Patentamts, Nr.12/82